# EUROPÄISCHE PATENTSCHRIFT

(11) **EP 0 804 236 B1**
(45) Veröffentlichungstag und Bekanntmachung des Hinweises auf die Patenterteilung: **02.04.2003**
(21) Anmeldenummer: 95906353.8
(22) Anmeldetag: 27.01.1995
(51) Int. Cl.: A61K 39/395

(54) **VERWENDUNG VON ANTI-TNF-ANTIKÖPERN ALS ARZNEIMITTEL ZUR BEHANDLUNG VON ERKRANKUNGEN MIT EINEM ERHÖHTEN INTERLEUKIN-6 SERUMSPIEGEL**
USE OF ANTI-TNF ANTIBODIES AS DRUGS IN TREATING DISEASES INVOLVING ELEVATED INTERLEUKIN-6 SERUM LEVELS
UTILISATION D'ANTICORPS ANTI-TNF DANS LA PREPARATION DE MEDICAMENTS POUR TRAITER DES MALADIES A TAUX SERIQUE ELEVE D'INTERLEUKINE-6

(30) Priorität: 07.02.1994 DE 4403669; 19.03.1994 DE 4409513
(43) Veröffentlichungstag der Anmeldung: 05.11.1997
(73) Patentinhaber: KNOLL AKTIENGESELLSCHAFT, 67061 Ludwigshafen (DE)
(72) Erfinder: STENZEL, Roswitha, D-68623 Lampertheim (DE); KAUL, Martin, D-67433 Neustadt (DE); DAUM, Lothar, D-67166 Otterstadt (DE); KEMPENI, Joachim, D-67433 Neustadt (DE); RAAB, Christa, D-67125 Dannstadt-Schauernheim (DE); SCHAEFER, Sibylle, D-69190 Walldorf (DE); EISELSTEIN, Jürgen, Weisenheim a. Berg 67273 (DE)
(74) Vertreter: Schweiger, Georg, Dr.
(86) Internationale Anmeldenummer: EP9500291
(87) Internationale Veröffentlichungsnummer: WO95020978

(56) Entgegenhaltungen:
- EP-A- 0 260 610
- EP-A- 0 351 789
- WO-A-92/16553
- CHEST, Bd. 101,Nr. 3, März 1992 CHICAGO, IL, VSA, Seiten 810-815, J-L. VINCENT ET AL. 'Administration of anti-TNF antibody improves left ventricular function in septic shock patients.'
- BLOOD, Bd. 83,Nr. 2, 15.Januar 1994 NEW YORK, NY, VSA, Seiten 446-451, T. VAN DER POLL ET AL. 'Differential effects of anti-tumor necrosis factor monoclonal antibodies on systemic inflammatory responses in experimental endotoxemia in chimpanzees.'
- CRITICAL CARE MEDICINE, Bd. 21,Nr. 3, März 1993 ANAHEIM, CA, VSA, Seiten 318-327, C. FISHER ET AL. 'Influence of an anti-tumor necrosis factor monoclonal antibody on cytokine levels in patients with sepsis.'
- CIRCULATORY SHOCK, Bd. 38,Nr. 2, Oktober 1992 NEW YORK, NY, VSA, Seiten 75-84, T. EMERSON ET AL. 'Efficacy of monoclonal antibody against tumor necrosis factor alpha in an endotoxemic baboon model.'
- THE JOURNAL OF EXPERIMENTAL MEDICINE, Bd. 170,Nr. 5, 1.November 1989 NEW YORK, NY, VSA, Seiten 1627-1633, Y. FONG ET AL. 'Antibodies to cachectin/tumor necrosis factor reduce interleukin 1beta and interleukin 6 appearance during lethal bacteremia.'
- CLINICAL RESEARCH, Bd. 42,Nr. 2, April 1994 THOROFARE, NJ, VSA, Seite 299a A. CHOW ET AL. 'Effect of monoclonal antibody to human tumor necrosis factor (TNF mAb) on TNFalpha, IL-1beta and IL-6 levels in patients with sepsis syndrome.'
- JOURNAL OF SURGICAL RESEARCH, Bd. 57,Nr. 5, November 1994 NEW YORK, NY, VSA, Seiten 625-631, P. MULLEN ET AL. 'Monoclonal antibody to tumor necrosis factor-alpha attenuates plasma interleukin-6 levels in porcine gram-negative sepsis.'

## Beschreibung

Die vorliegende Erfindung betrifft die Verwendung von anti-TNF-Antikörpern bei der Behandlung von Erkrankungen mit einem erhöhten Interleukin-6 Serumspiegel.

Es ist bekannt, daß der Begriff Tumor Nekrose Faktor (TNF) zwei zytotoxische Faktoren (TNF-α und TNF-β) umfaßt, die größtenteils von aktivierten Lymphozyten und Monozyten gebildet werden.

In der EP 260 610 werden beispielsweise anti-TNF-Antikörper beschrieben, die bei Krankheiten, die mit einer Erhöhung von TNF im Blut verbunden sind, wie septischer Schock, Transplantatabstoßung, Allergien, Autoimmunkrankheiten, Schocklunge, Blutgerinnungsstörungen oder entzündlichen Knochenerkrankungen zur Inaktivierung von TNF einsetzbar sein sollen.

Erkrankungen, die durch erhöhte Interleukin-6 Serumspiegel bei Patienten gekennzeichnet sind, sind beispielsweise Folgeerscheinungen nach Transplantationen, Autoimmunerkrankungen und insbesondere bestimmte Formen der Sepsis.

In medizinischen Lehrbüchern wird Sepsis als ein klinischer Sammelbegriff für Zustände definiert, bei denen - ausgehend von einem Herd - bakterielle Erreger in die Blutbahn gelangen, wodurch ein großes Spektrum subjektiver und objektiver Krankheitserscheinungen ausgelöst wird. Weiterhin wird festgestellt, daß in Abhängigkeit vom Erregertyp, der Reaktionslage des Organismus, dem Primärherd und den wechselnden Organbeteiligungen das Krankheitsbild sehr variieren kann (Sturm et al. "Grundbegriffe der Inneren Medizin", 13. Auflage, Seite 570, Gustav Fischer Verlag, Stuttgart, 1984).

Für den komplexen pathophysiologischen Ablauf einer Sepsis wird die Beteiligung einer Reihe von Cytokinen diskutiert. Besonders TNF wird eine wichtige Rolle beim septischen Schock aufgrund tierexperimenteller Daten zugeschrieben (Beutler et al., Science 229, 869-871, 1985).

Dies hat letztlich dazu geführt, daß klinische Studien zur Behandlung von Sepsispatienten mit anti-TNF-Antikörpern durchgeführt wurden.

In einer vor kurzem veröffentlichten Mehrzentren-Phase II- Studie zur Behandlung von schwerer Sepsis mit einem murinen monoklonalen anti-TNF-Antikörper wurde allerdings gefunden, daß das Gesamtkollektiv (80 Patienten) hinsichtlich Überlebensrate nicht von der Behandlung mit dem Antikörper profitierte. Lediglich die Patienten mit erhöhten zirkulierenden TNF-Konzentrationen schienen von hochdosierter anti-TNF-Antikörper-Gabe hinsichtlich der Überlebenswahrscheinlichkeit zu profitieren (C.J.Fisher et al., Critical Care Medicine, Vol. 21, No.3, Seite 318-327). Weiterhin wird in dieser Studie auf eine Korrelation der Plasmawerte von TNF und Il-6 hingewiesen.

Die Rolle, die das Cytokin Interleukin-6 (Il-6) bei der Sepsis spielt, ist unklar und widersprüchlich. Bei einigen Sepsispatienten wurden erhöhte Serumspiegel an Il-6 gefunden (Hack et al., Blood 74, Nr.5, 1704-1710, 1989).

Waage beschreibt, daß die Konzentrationen der Cytokine Il-6 und Il-8 mit der Schwere des Schocks korrelieren; daß sie aber nicht, weder allein noch in Kombination mit TNF, die Entwicklung eines Schocksyndroms hinsichtlich Lethalität beeinflussen (Waage in "Tumor Necrosis Factors",ed. B.Beutler, Raven Press, New York,1992, Seite 275-283).

Von einigen Wissenschaftlern wird Il-6 eine günstige Rolle beim septischen Schock zugeschrieben, da Il-6 in Form einer negativen Feedback-Kontrolle die LPS-induzierte TNF Produktion hemmt (Libert et al. in "Tumor Necrosis Factor: Molecular and Cellular Biology and Clinical Relevance", ed. W. Fiers, Karger, Basel, 1993, Seite 126-131).

Überraschenderweise wurde nun gefunden, daß sich TNF-Antagonisten besonders erfolgreich als Arzneimittel zur Behandlung von Erkrankungen einsetzen lassen, die durch erhöhte Interleukin-6 Serumspiegel gekennzeichnet sind.

Die Behandlung von Sepsis mit TNF-Antagonisten ist gemäß dieser Erfindung besonders erfolgreich ,beispielsweise gemessen an einer deutlichen Reduzierung der Sterblichkeit, wenn solche Sepsispatienten behandelt werden, die bei Beginn der Behandlung Il-6 Werte von 1000 pg/ml und mehr aufweisen.

Unter erhöhten Il-6 Serumspiegeln sind solche Werte zu verstehen, die gegenüber physiologischen Serumspiegeln bei gesunden Probanden mindestens zehnfach erhöht sind.

Es wurden Serumkonzentrationen von Il-6 bei Sepsispatienten beobachtet,die bis zu 20000 fach über den Werten von gesunden Probanden lagen.

Die "normalen" Il-6 Serumwerte liegen üblicherweise unterhalb der Nachweisgrenze, was je nach verwendetem Testsystem leicht variieren kann. Sie liegen jedoch höchstens bei 20 pg/ml.

Die Serumkonzentrationen an Il-6 lassen sich mit üblichen Nachweisverfahren wie RIA oder ELISA bestimmen. Ein gut geeignetes Nachweissystem ist beispielsweise das "IL-6-EASIA" der Fa. Medgenix.

Die Konzentration an Il-6 kann auch über einen Aktivitätstest, bei dem beispielsweise C-reaktives Protein getestet wird, bestimmt werden.

Als TNF-Antagonisten geeignet sind anti-TNF-Antikörper, TNF-Rezeptoren und lösliche Fragmente davon, TNF-Bindeproteine oder solche TNF-Derivate, die noch TNF-Rezeptorbindung besitzen, aber keine TNF-Aktivität mehr aufweisen. Solche TNF-Antagonisten sind dadurch gekennzeichnet, daß sie bereits gebildetes TNF abfangen und nicht an den TNF-Rezeptor gelangen lassen oder daß sie mit TNF um den Rezeptor konkurrieren.

Geeignet für die erfindungsgemäße Verwendung sind aber auch TNF-Antagonisten, die die Bildung oder Freisetzung von TNF verhindern. Solche Substanzen inhibieren beispielsweise die Genexpression von TNF oder die Freisetzung von TNF aus Vorläuferformen.

Solche TNF-antagonistischen Aktivitäten sind beispielsweise von Xanthinderivaten, Glucocorticoiden, Prostaglandin E 2, Thalidomid, Interleukin-4, Interleukin-10, Granulozyten Stimulating Factor (G-CSF), Cyclosporin, α-Antitrypsin beschrieben. Daher sind auch solche Verbindungen als TNF-Antagonisten geeignet.

Besonders bevorzugt für die erfindungsgemäße Verwendung sind anti-TNF-Antikörper.

Die zur erfindungsgemäßen Verwendung geeigneten anti-TNF-Antikörper sind bekannt (EP 260 610, EP 351 789, EP 218 868). Es können sowohl polyklonale als auch monoklonale Antikörper verwendet werden. Weiterhin sind auch TNF-bindende Antikörperfragmente wie Fab- oder F(ab')₂-Fragmente oder single-chain-Fv-Fragmente geeignet.

Ferner sind auch humanisierte oder humane anti-TNF-Antikörper oder deren TNF-bindende Fragmente gut geeignet, da diese Moleküle in menschlichen Patienten keine anti-Maus-Antigenizität verursachen sollten.

Es können auch Gemische von verschiedenen anti-TNF-Antikörpern oder von anti-TNF-Antikörpern und TNF-Rezeptor-Fragmenten als Wirkstoff verwendet werden.

Zur vorliegenden Erfindung gehören pharmazeutische Zubereitungen, die neben nicht-toxischen, inerten pharmazeutisch geeigneten Trägerstoffen die anti-TNF-Antikörper enthalten, sowie Verfahren zur Herstellung dieser Zubereitungen.

Die Formulierung der anti-TNF-Antikörper geschieht in für biotechnisch hergestellte Wirkstoffe üblicher Weise, in der Regel als Flüssigformulierung oder Lyophilisat (siehe z.B. Hagers Handbuch der pharmazeutischen Praxis, Bd. 2, 5. Auflage, 1991, S. 720, ISBN 3-540-52459-2). Die Herstellung der oben aufgeführten pharmazeutischen Zubereitungen erfolgt in üblicher Weise nach bekannten Methoden, z.B. durch Mischen des oder der Wirkstoffe mit dem oder mit den Trägerstoffen.

Im allgemeinen hat es sich als vorteilhaft erwiesen, den oder die für die erfindungsgemäße Verwendung geeigneten Wirkstoffe in Gesamtmengen von etwa 0,1 bis etwa 1000, vorzugsweise 0,1 bis 10 mg/kg Körpergewicht je 24 Stunden, ggf. in Form mehrerer Einzelgaben oder als Dauerinfusion und ggf. über eine Therapiedauer von mehreren Tagen hinweg zur Erzielung der gewünschten Ergebnisse zu verabreichen. Die Applikation kann als intravenöse Kurzinfusion der Einzelgaben oder als kontinuierliche Langzeitinfusion der Tagesdosis über 24 Stunden erfolgen. Eine Einzelgabe enthält den oder die Wirkstoffe vorzugsweise in Mengen von etwa 0,1 bis etwa 10 mg/kg Körpergewicht. Es kann jedoch erforderlich sein, von den genannten Dosierungen abzuweichen und zwar in Abhängigkeit von Alter und Größe des zu behandelnden Patienten sowie der Art und der Schwere der zugrundeliegenden Erkrankung, der Art der Zubereitung und der Applikation des Arzneimittels sowie dem Zeitraum bzw. Intervall, innerhalb welchem die Verab- reichung erfolgt. Die Erfindung ist in dem folgenden Beispiel weiter veranschaulicht.

### Beispiel

Behandlung von Sepsispatienten mit einem murinen anti-TNF-Antikörperfragment (F(ab')₂).

In einer multizentrischen klinischen Studie wurden insgesamt 122 Patienten mit schwerer Sepsis mit anti-TNF-Antikörperfragment in verschiedenen Dosierungen bzw. mit Placebo behandelt.

Die vier untersuchten Therapieverfahren unterschieden sich lediglich in der Höhe der Einzeldosis des anti-TNF-Antikörperfragments voneinander. Diese war entweder 0,1 mg/kg Körpergewicht, 0,3 mg/kg Körpergewicht oder 1,0 mg/kg Körpergewicht. Die Patienten der vierten Gruppe erhielten zum Vergleich eine "Scheintherapie" (Placebo). Dabei wurdem die Patienten nach einem Zufallsprinzip einem der vier beschriebenen Therapieregime mit anti-TNF-Antikörperfragment zugeordnet. Die beschriebene Therapie, die zusätzlich zur Standardtherapie bei septischen Patienten gegeben wurde, wurde nach Diagnosestellung (= Erfüllung der Einschlußkriterien der Studie) insgesamt neunmal (9 x) im Abstand von jeweils 8 Stunden (also über drei Tage hinweg) als Kurzinfusion appliziert. Insgesamt wurden 122 Patienten in die Studie eingeschlossen, wovon 34 Patienten der 0,1 mg/kg-Dosisgruppe, 30 Patienten der 0,3 mg/kg-Dosisgruppe, 29 Patienten der 1,0 mg/kg-Dosisgruppe und 29 Patienten der Placebogruppe zugeordnet wurden.

Von den 122 Patienten konnte bei 119 Patienten Il-6 Serumkonzentrationen vor Therapiebeginn gemessen werden. Bei 36 wurden Il-6 Serumspiegel > 1000 pg/ml gemessen, bei 83 Patienten Serumspiegel < 1000 pg/ml.

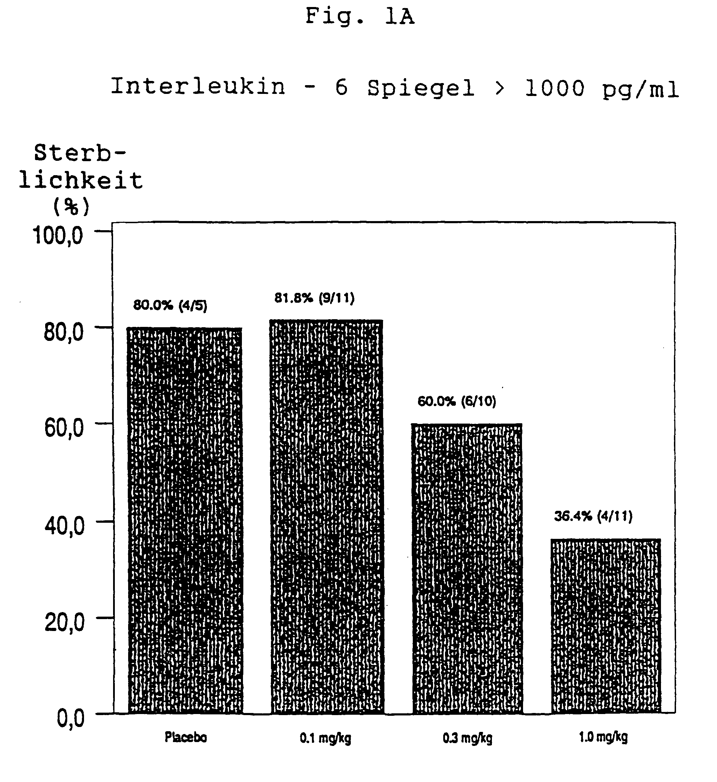

Fig. 1A zeigt die Sterblichkeit im Kollektiv Il-6 > 1000 pg/ml bei den verschiedenen Behandlungsgruppen (Placebo, 0,1 ; 0,3 und 1,0 mg Antikörper pro kg Körpergewicht).

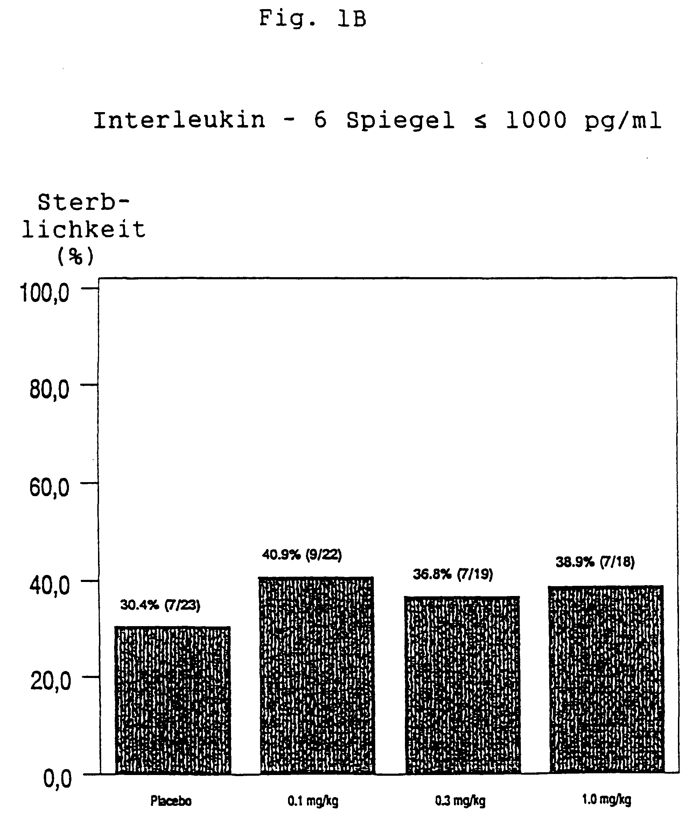

Fig. 1B zeigt die Sterblichkeit im Kollektiv Il-6 < 1000 pg/ml bei den verschiedenen Behandlungsgruppen (Placebo, 0,1 ; 0,3 und 1,0 mg Antikörper pro kg Körpergewicht).

Bei den Patienten mit Il-6 >1000 pg/ml wurde durch die Behandlung mit anti-TNF-Antikörperfragment eine dosisabhängige Reduzierung der Sterblichkeit von 80,0% (=Placebogruppe) auf 36,4% (1,0 mg/kg Antikörper) erreicht (Fig. 1A).

Bei den Patienten mit Il-6 < 1000 pg/ml wurde durch die Behandlung mit anti-TNF-Antikörperfragment die Sterblichkeit nicht verringert sondern im Gegenteil noch geringfügig erhöht (30,4% in der Placebogruppe gegenüber 38,9% in der Gruppe mit 1,0 mg/kg Antikörper) - (Fig. 1B).

Das Ergebnis dieser klinischen Studie belegt deutlich, daß die Behandlung von schwerer Sepsis mit anti-TNF-Antikörpern nur dann erfolgreich verläuft, wenn solche Sepsispatienten behandelt werden, die einen Il-6 Serumspiegel von >1000 pg/ml besitzen; die Behandlung von Patienten mit Il-6 Serumspiegel von <1000 pg/ml ist ohne Erfolg und manchmal sogar kontraindiziert.

## Patentansprüche

1. Verwendung von TNF-Antagonisten zur Herstellung von Arzneimitteln zur Behandlung von Sepsis bei Patienten, die bei Beginn der Behandlung Interleukin 6-Serumspiegel von größer 1000 pg/ml aufweisen.

2. Verwendung nach Anspruch 1, **dadurch gekennzeichnet, daß** die TNF-Antagonisten monoklonale anti-TNF-Antikörper oder TNF bindende Antikörperfragmente sind.

3. Verwendung nach Anspruch 2, **dadurch gekennzeichnet, daß** die anti-TNF-Antikörper humanisierte oder humane anti-TNF-Antikörper sind.

4. Verwendung nach Anspruch 1, **dadurch gekennzeichnet, daß** die TNF-Antagonisten TNF-Rezeptoren, lösliche Fragmente davon oder TNF-Bindeproteine sind.

## Revendications

1. Utilisation d'antagonistes du TNF pour la préparation de médicaments destinés au traitement de la septicémie chez des patients qui présentent au début du traitement des taux sériques dinterleukine 6 supérieurs à 1000 pg/ml.

2. Utilisation selon la revendication 1, **caractérisée en ce que** les antagonistes du TNF sont des anticorps monoclonaux anti-TNF ou des fragments d'anticorps fixant le TNF.

3. Utilisation selon la revendication 2, **caractérisée en ce que** les anticorps anti-TNF sont des anticorps anti-TNF humanisés ou humains.

4. Utilisation selon la revendication 1, **caractérisée en ce que** les antagonistes du TNF sont des récepteurs du TNF, des fragments solubles de ceux-ci ou des protéines de fixation du TNF.

## Claims

1. The use of TNF antagonists for the manufacture of medicaments for the treatment of sepsis in patients who have interleukin 6 serum levels of more than 1000 pg/ml at the start of treatment.

2. The use according to Claim 1, **characterised in that** the TNF antagonists are monoclonal anti-TNF antibodies or TNF-binding antibody fragments.

3. The use according to Claim 2, **characterised in that** the anti-TNF antibodies are humanised or human anti-TNF antibodies.

4. The use according to Claim 1, **characterised in that** the TNF antagonists are TNF receptors, soluble fragments thereof or TNF binding proteins.
